# EUROPÄISCHE PATENTSCHRIFT

(11) **EP 0 614 908 B1**
(45) Veröffentlichungstag und Bekanntmachung des Hinweises auf die Patenterteilung: **07.05.1997**
(21) Anmeldenummer: 94102994.4
(22) Anmeldetag: 28.02.1994
(51) Int. Cl.: C07J 41/00, C07J 43/00, C07J 9/00

(54) **Verfahren zur Herstellung von 3beta-Aminocholansäurederivaten**
Method of preparation of 3-beta-aminocholanic acid derivatives
Procédé de préparation des dérivés de l'acide 3-bêta-aminocholanique

(30) Priorität: 09.03.1993 DE 4307305
(43) Veröffentlichungstag der Anmeldung: 14.09.1994
(73) Patentinhaber: HOECHST AKTIENGESELLSCHAFT, 65929 Frankfurt am Main (DE)
(72) Erfinder: Enhsen, Alfons, Dr., D-64572 Büttelborn (DE); Glombik, Heiner, Dr., D-65719 Hofheim am Taunus (DE); Kramer, Werner, Dr. Dr., D-55130 Mainz (DE); Wess, Günther, Dr., D-63526 Erlensee (DE)

(56) Entgegenhaltungen:
- EP-A- 0 489 423
- CHEMICAL ABSTRACTS, vol. 106, no. 11, 16.März 1987 Columbus, Ohio, US; abstract no. 84937, A. BELLINI 'Regioselective Amination of 5-beta-Cholanic Acids' Seite 640; Spalte 1;
- CHEMICAL ABSTRACTS, vol. 108, no. 21, 23.Mai 1988 Columbus, Ohio, US; abstract no. 187049, NGUYEN THI THU PHONG 'Synthesis of Funtumine from Pregnenolone' Seite 738; Spalte 2;
- SYNTHESIS, Nr. 1, Januar 1981 STUTTGART DE, Seiten 1-28, O. MITSUNOBU 'The Use of Diethyl Azodicarboxylate and Triphenyphosphine in Synthesis and Transformation of Natural Products'

## Beschreibung

3β-Aminocholansäureester gewinnen zunehmend an Bedeutung als wertvolle Zwischenprodukte zur Herstellung von Verbindungen für eine Reihe pharmazeutischer Anwendungen. Sie werden z. B. als Synthesebausteine zur Herstellung von Wirkstoff-Gallensäurekonjugaten eingesetzt (EP-A-0 417 725 = U.S. Patentanmeldung Nr. 07/806,799). Der Gallensäurerest fungiert in diesen Konjugaten als Carrier und erlaubt einen leberselektiven Transport des kovalent gebundenen Wirkstoffs über den enterohepatischen Kreislauf. 3β-Aminocholansäureester finden ebenfalls Verwendung als wichtige Synthone bei der Synthese von Gallensäureresorptionsinhibitoren (vergl. z. B. EP-A-0 489 423 = U.S. Patentanmeldung Nr. 07/802,413). Diese Gallensäurederivate sind selektive Inhibitoren des intestinalen Gallensäuretransportsystems und führen daher zu einer Unterbrechung des enterohepatischen Kreislaufs und damit zu einer Senkung des Plasmacholesterinspiegels.

In der Literatur sind mehrere Verfahren zur Synthese von 3-Aminocholansäurederivaten der Formel I beschrieben, 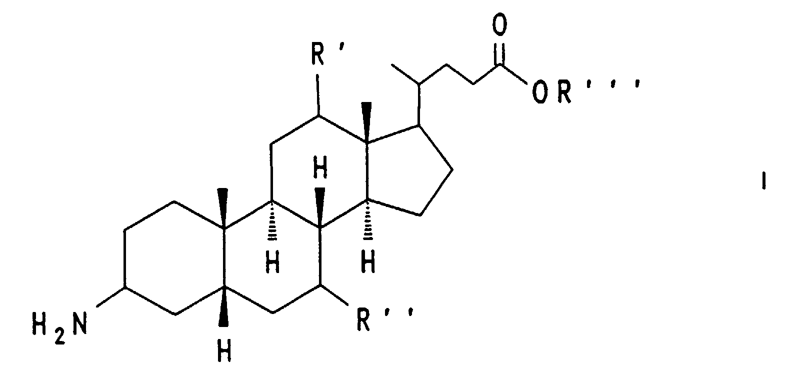 worin R', R'', R''' z. B. die in den nachfolgenen angegebenen Literaturstellen angegebenen Bedeutungen haben.

So wird aus 3-Ketocholansäuren das entsprechende Oxim dargestellt. Die Oximfunktion kann mit Natrium/Amylalkohol (Redel et al., Bull. Soc. Chim. Fr. 877, (1949)) oder unter katalytischen Bedingungen mit Wasserstoff reduziert werden (Satoh, Bull. Chem. Soc. Jap., 1965, 38, 1581). Nachteil dieser Verfahren ist, daß in allen Fällen isomere Gemische der 3α- und 3β-Aminocholansäurederivate der Formel I erhalten werden, die aufwendig getrennt und gereinigt werden müssen. Die Stereoselektivität variiert mit den Substraten und den Reaktionsbedingungen, weiterhin liefern diese Verfahren nur mäßige Ausbeuten.

Entsprechend einem ebenfalls in EP-489,423 beschriebenen Verfahren wird nach Aktivierung der 3-Hydroxyfunktion von 3-Hydroxycholansäureestern, z. B. durch Umsetzung mit Methansulfonsäurechlorid, durch nukleophile Substitution mit Natriumazid das 3-Azidocholansäurederivat erhalten.

Die Reaktion verläuft stereochemisch eindeutig. Anschließende Reduktion der Azidogruppe führt zu 3-Aminocholansäureestern. Die Reaktionsführung beim Azidaustausch ist jedoch problematisch, da bei diesem Verfahren Azidverbindungen längere Zeit hohen Temperaturen (von ca. 130°C) ausgesetzt sind. Weiterhin liegen die Ausbeuten nur im Bereich von 30 bis 50 %.

Es wurde nun überraschenderweise gefunden, daß die Verbindungen der Formel II aus einfach verfügbaren Ausgangsverbindungen in hohen Ausbeuten stereoselektiv und nach einem sicheren Verfahren hergestellt werden können. Somit werden Schlüsselbausteine zur Synthese wertvoller Arzneimittel auf wirtschaftlichem Weg zur Verfügung gestellt.

Die Erfindung betrifft ein Verfahren zur Herstellung von 3β-Aminocholansäurederivaten der Formel II, 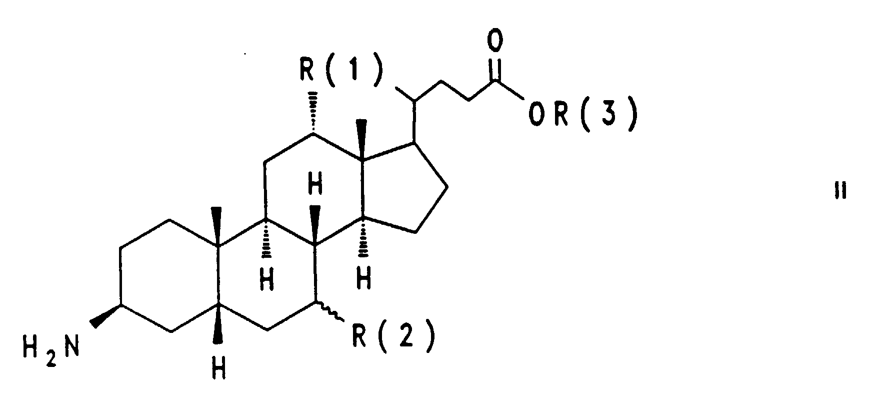 worin
- R(1) =: H, OH,
- R(2) =: H, α-OH oder β-OH bedeuten
und
- R(3): ein unverzweigter C₁-C₄-Alkylrest, ein verzweigter C₃-C₄-Alkyl- oder ein Benzylrest ist,
und von deren Salzen mit Mineralsäuren,
welches dadurch gekennzeichnet ist, daß man
a) einen 3α-Hydroxycholansäureester der Formel III 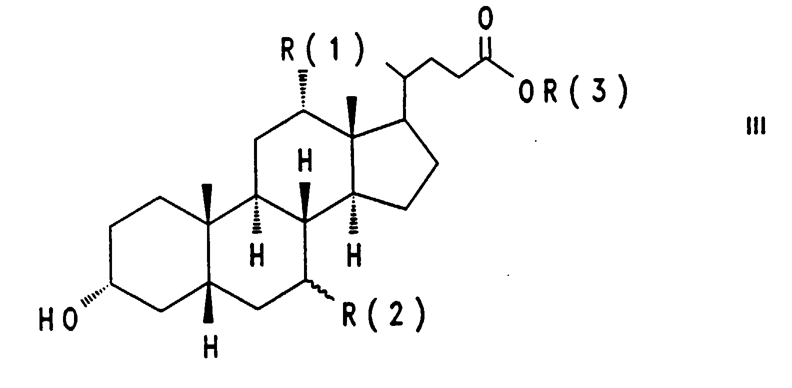 mit Phthalimid zum 3β-Phthalimidoderivat der Formel IV 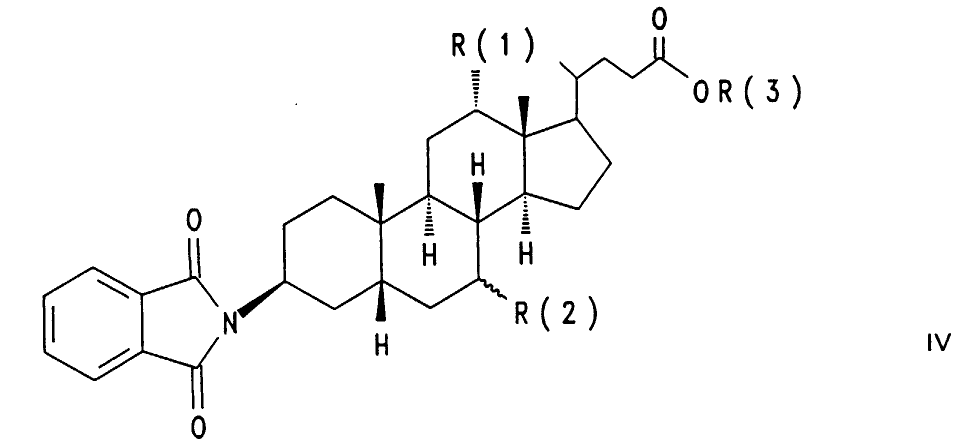 umsetzt,
b) einen erhaltenen 3β-Phthalimidocholansäureester der Formel IV mit Hydrazinhydrat oder Phenylhydrazin zur Reaktion bringt, und
c) durch Behandlung des Reaktionsproduktes mit einer wäßrigen Lösung einer Mineralsäure die Phthalimidgruppe spaltet, wobei ein Säureadditionssalz der Verbindung II mit der Mineralsäure entsteht, und
d) gegebenenfalls ein erhaltenes Salz in die freie Verbindung überführt.

Die Umsetzung von Verbindung III mit Phthalimid wird zweckmäßigerweise unter den Bedingungen der Mitsunobu-Reaktion (Mitsunobu, Synthesis 1, 1981; Org. React. Vol. 42, 335 (1992)) durchgeführt in Gegenwart eines geeigneten Phosphins, bevorzugt Triphenylphosphin, und Azodicarbonsäure-diethyl-(oder diisopropyl)-ester. Die Durchführung erfolgt zweckmäßigerweise in einem geeigneten Lösungsmittel, wie Tetrahydrofuran oder auch Dioxan, bei Temperaturen von 20 bis 50°C.

Die freie Verbindung der Formel II erhält man nach üblichen Methoden aus den Salzen.

Der Vorteil dieser Reaktion besteht darin, daß keine Schutzgruppen für Hydroxygruppen in den Positionen 7 oder 12 erforderlich sind, die Reaktion ist regioselektiv und findet nur in der 3-Position statt, weiterhin ist sie diastereoselektiv, es entsteht aus der 3α-Hydroxygruppe des Steroids ausschließlich das 3β-Phthalimidderivat in hohen Ausbeuten.

Mineralsäuren sind z. B. Schwefelsäure, Salpetersäure und Salzsäure. Bei dem erfindungsgemäßen Verfahren wird vorzugsweise eine wäßrige Lösung von Salzsäure verwendet.

Ester der Formel III werden aus den entsprechenden Säuren nach üblichen Verfahren hergestellt.

Herstellung der Ausgangsverbindungen der Formel III am Beispiel von Cholansäuremethylester (Fieser et al., J. Am. Chem. Soc. 74, 1952, 3309)

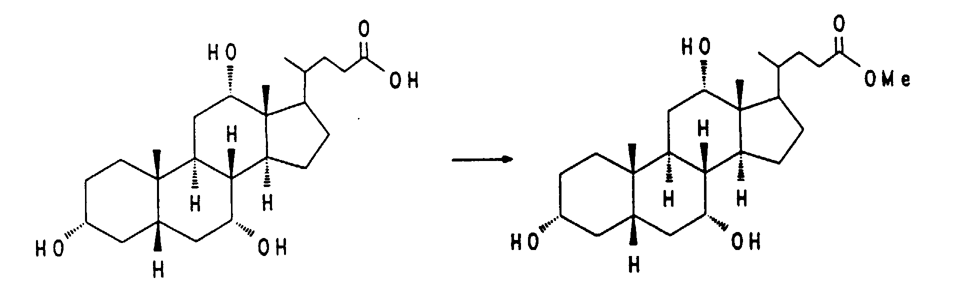

Zu 4,5 l Methanol werden unter Eiskühlung langsam 450 ml Acetylchlorid zugetropft, so daß die Temperatur 10 bis 15°C nicht übersteigt. Nach der Zugabe wird noch 15 Minuten bei ca. 10°C nachgerührt. Anschließend werden bei ca. 5°C 600 g (1,47 mol) Cholsäure eingetragen. Das Eisbad wird entfernt, und nach einer weiteren Stunde unter Rühren ist die Reaktion beendet. Das Reaktionsgemisch wird langsam zu 12 l Wasser zugegeben. Es wird noch 2 Stunden gerührt, anschließend abgesaugt und mit 3 l Wasser nachgewaschen. Die kristalline Substanz wird bei 50°C im Vakuum getrocknet. Man erhält 610 g (98 %) Cholsäuremethylester (Schmp. 154°C).

Die Erfindung wird durch die nachfolgenden Beispiele näher erläutert:

### Beispiel 1

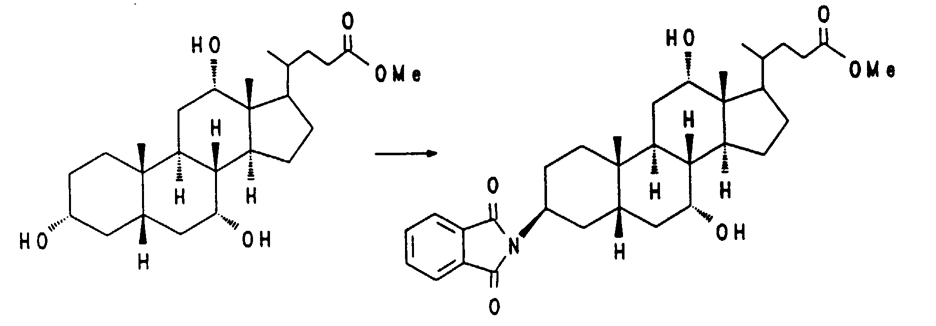

Ein Gemisch aus 500 g (1,18 mol) Cholsäuremethylester, 341 g (1,30 mol) Triphenylphosphin und 191 g (1,29 mol) Phthalimid wird in 3,0 l absolutem THF auf 40°C erwärmt. Unter Rühren und unter Schutzgas wird langsam eine Lösung aus 400 ml (2,57 mol) Azodicarbonsäurediethylester und 200 ml THF zugetropft, so daß die Temperatur 40 bis 50°C nicht übersteigt (leichte Kühlung erforderlich). Nach der Zugabe wird noch 1,5 Stunden bei ca. 45°C gerührt. Das Reaktionsgemisch wird eingeengt*. Der Rückstand wird in 15 l Isopropanol zum Sieden erhitzt und gerührt bis alle Bestandteile fein verteilt sind. Das Gemisch wird 48 Stunden stehengelassen, abgesaugt und mit 10 l Isopropanol gewaschen. Nach Trocknung im Vakuum bei 60°C werden 527 g (81 %) Produkt erhalten. Die Verbindung kann ohne weitere Reinigung zur nächsten Stufe umgesetzt werden*. (Nach Einengung kann das Rohprodukt auch über Kieselgel chromatographiert werden).
R_{f} = 0,61, Laufmittel: Chloroform/MeOH 95:5
MS (FAB, 3-NBA/LiCl) C₃₃H₄₅NO₆ (551): 558 (M+Li⁺)

### Beispiel 2

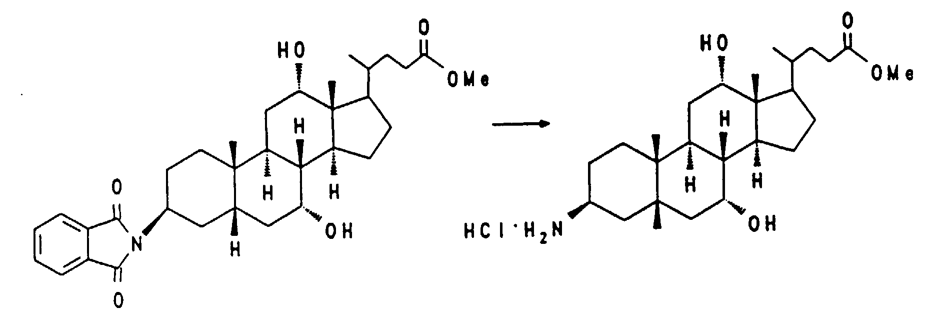

200 g (0,36 mol) Beispiel 1 werden in 1,6 l Methanol gelöst, mit 60 ml 80 %igem Hydrazinhydrat versetzt und 3 Stunden unter Rückfluß erhitzt. Das Reaktionsgemisch wird auf 40°C abgekühlt und es werden zügig 480 ml 2N Salzsäure zugegeben, bis ein pH-Wert von ca. 2 bis 3 erreicht ist. Nach weiteren 30 Minuten bei 40°C wird auf -5°C abgekühlt und von den Nebenprodukten abgesaugt. Die erhaltene Lösung wird mit Natriumhydrogencarbonat (ca. 35 g) auf pH 6 bis 7 gebracht. Anschließend wird eingeengt bis das Gesamtvolumen noch ca. 500 ml beträgt. Der Rückstand wird abgesaugt, zweimal mit je 500 ml Wasser, dann zweimal mit je 500 ml Essigester gewaschen und im Trockenschrank bei 60°C getrocknet. Die Ausbeute beträgt 117 g (70 %).
R_{f} = 0,28, Laufmittel: CHCl₃/MeOH 7:3, 5 % NEt₃
MS (FAB, 3-NBA/LiCl) C₂₅H₄₃NO₄ (421): 428 (M+Li⁺)

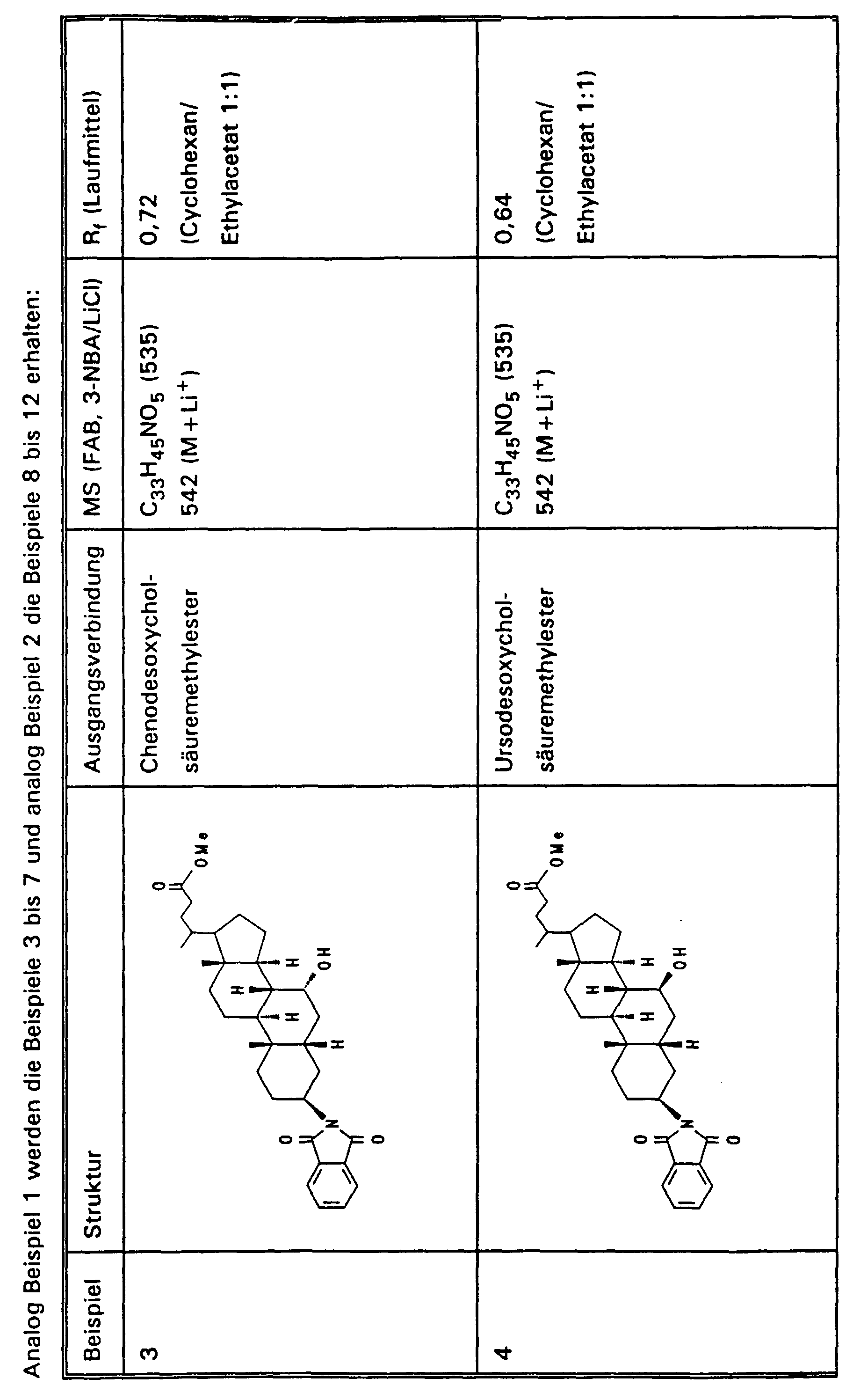

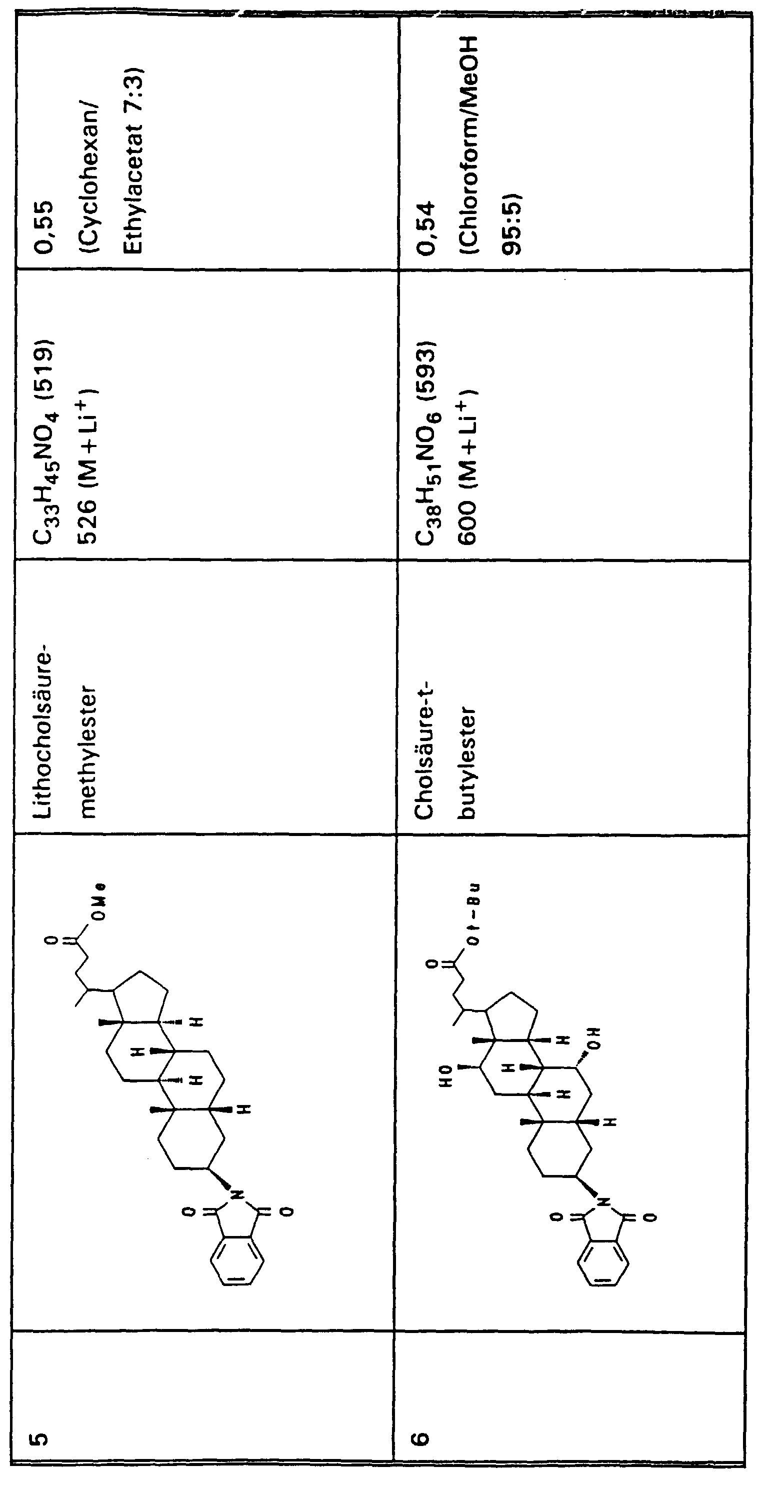

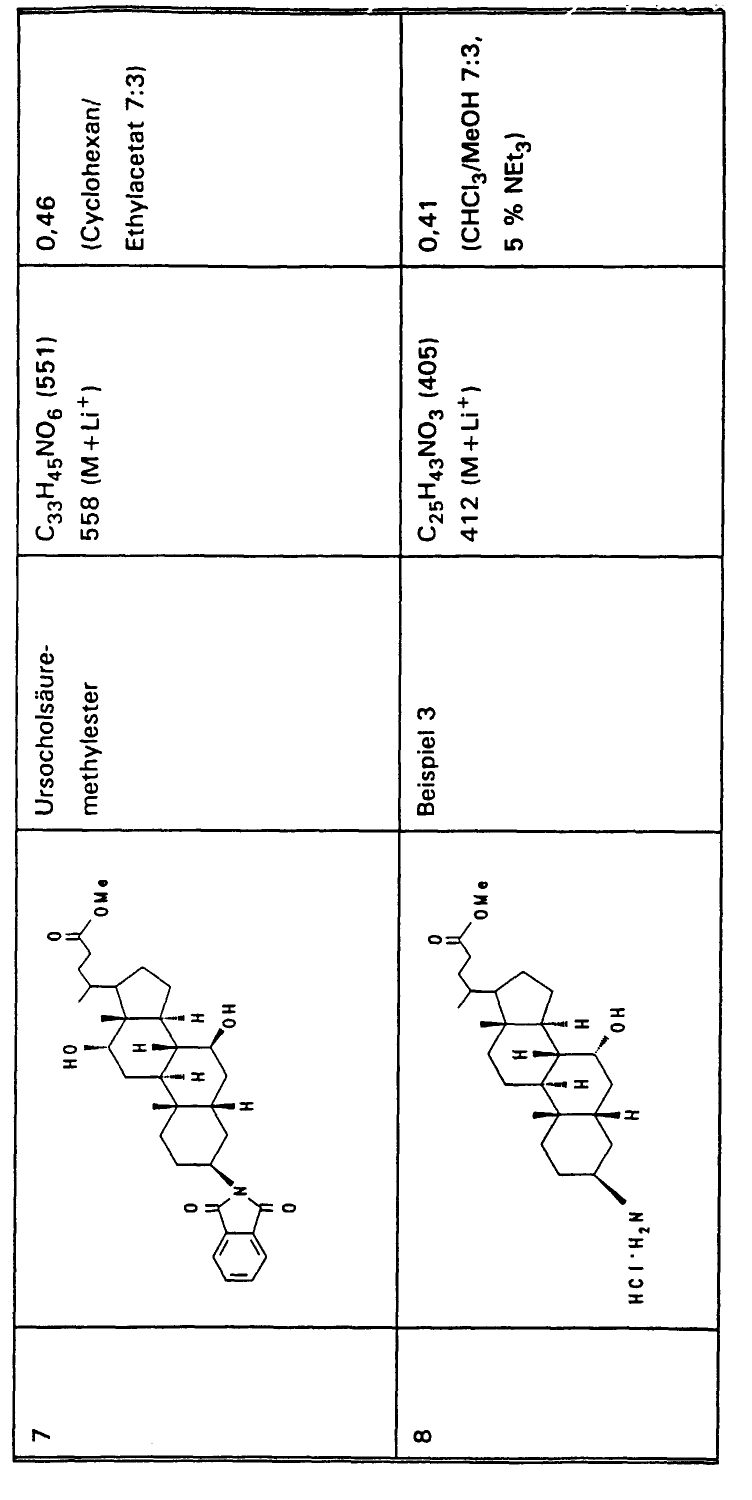

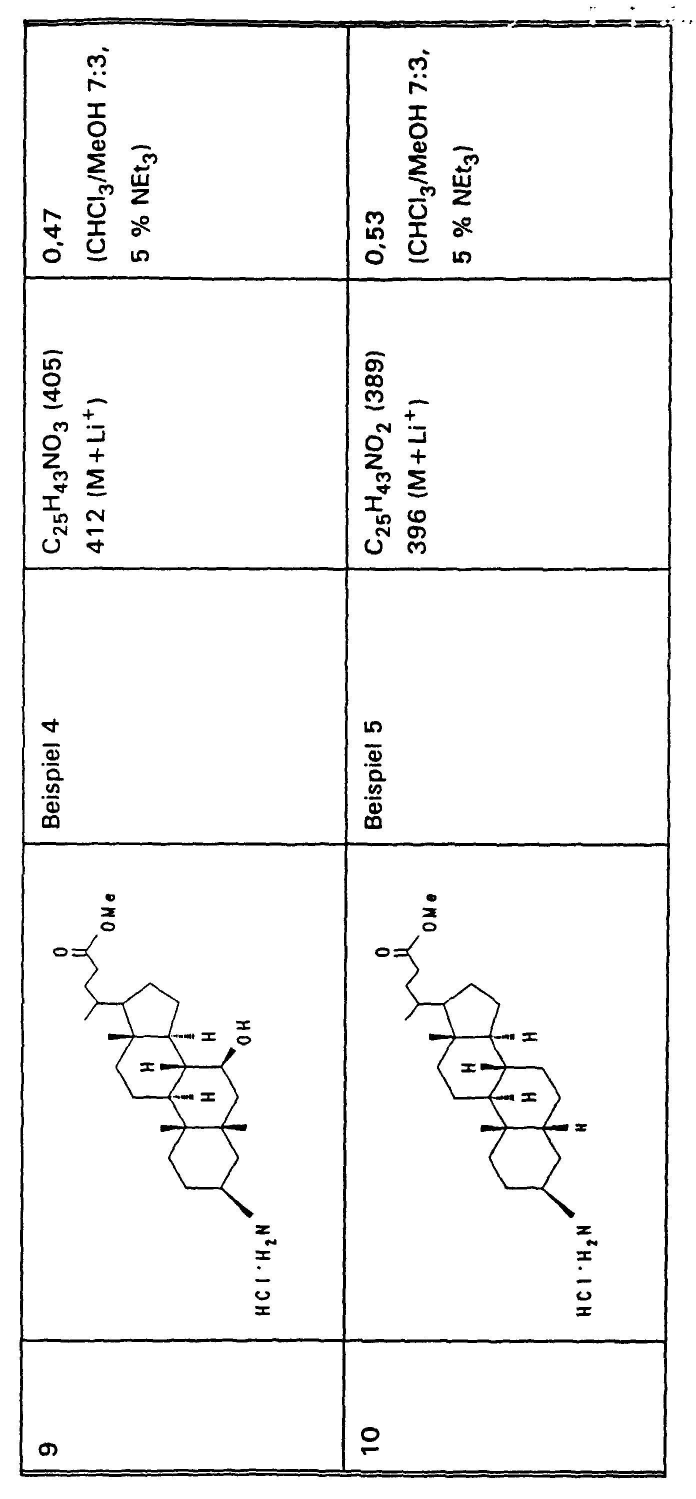

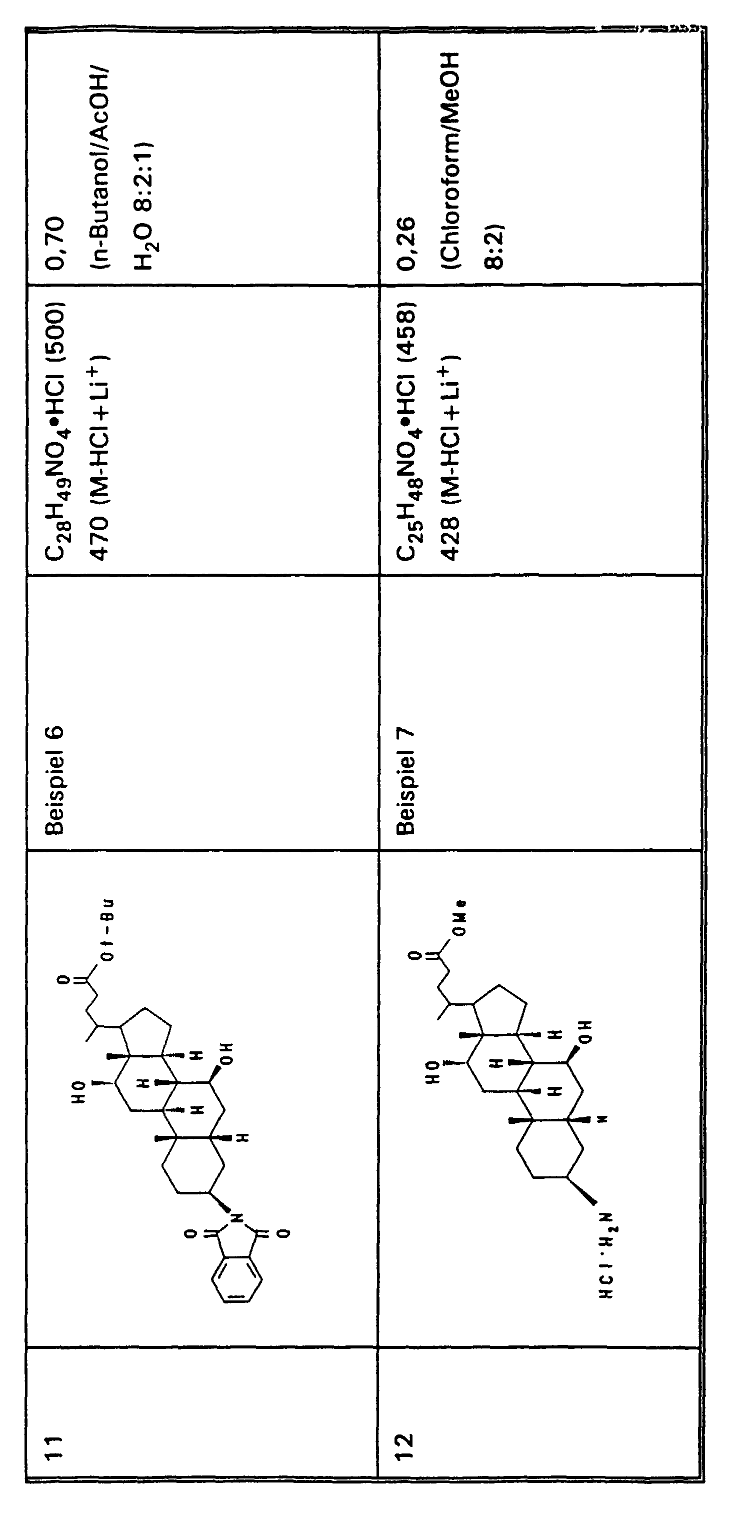

## Patentansprüche

1. Verfahren zur Herstellung von 3β-Aminocholansäurederivaten der Formel II, 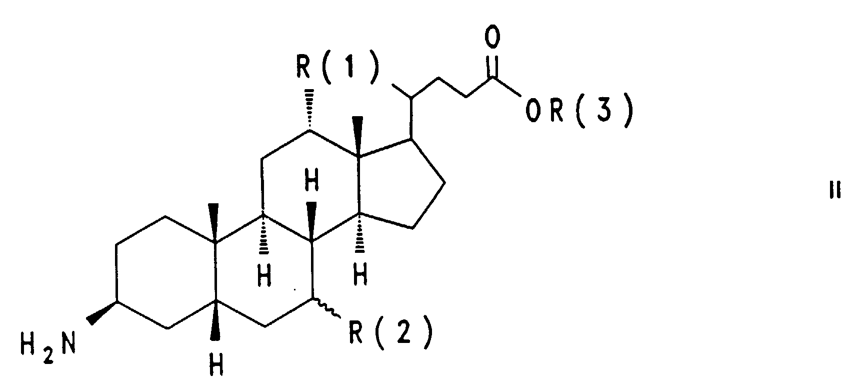 worin
R(1) = H, OH,
R(2) = H, α-OH oder β-OH bedeuten
und
R(3) ein unverzweigter C₁-C₄-Alkylrest, ein verzweigter C₃-C₄-Alkyl- oder ein Benzylrest ist,
und von deren Salzen mit Mineralsäuren,
dadurch gekennzeichnet, daß man
a) einen 3α-Hydroxycholansäureester der Formel III 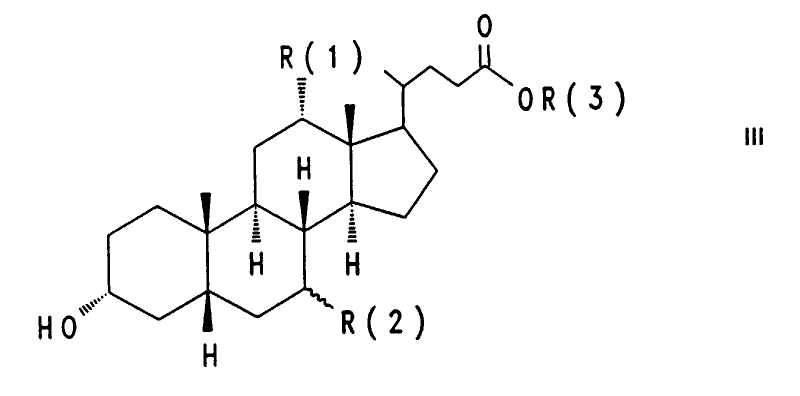 mit Phthalimid zum 3β-Phthalimidoderivat der Formel IV 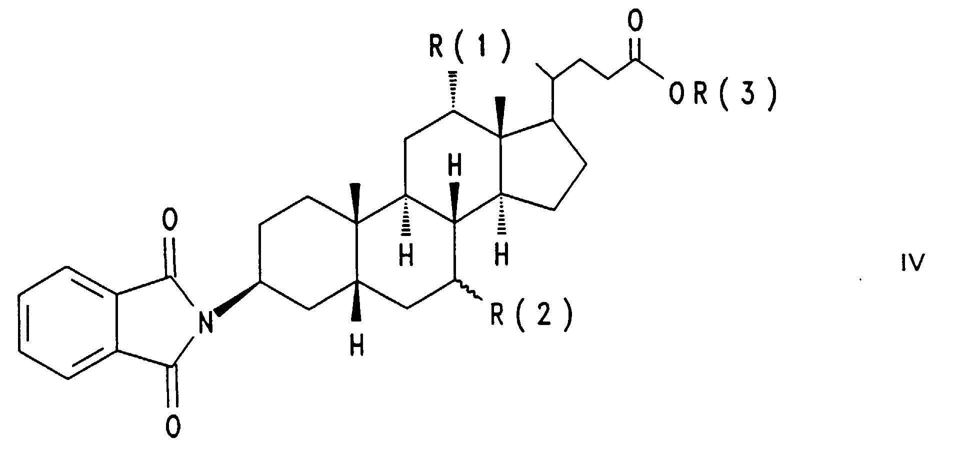 umsetzt,
b) einen erhaltenen 3β-Phthalimidocholansäureester der Formel IV mit Hydrazinhydrat oder Phenylhydrazin zur Reaktion bringt, und
c) durch Behandlung des Reaktionsproduktes mit einer wäßrigen Lösung einer Mineralsäure die Phthalimidgruppe spaltet, wobei ein Säureadditionssalz der Verbindung II mit der Mineralsäure entsteht, und
d) gegebenenfalls ein erhaltenes Salz in die freie Verbindung der Formel II überführt.

2. Verfahren gemäß Anspruch 1, dadurch gekennzeichnet, daß man die Umsetzung von Verbindungen der Formel III mit Phthalimid in Gegenwart eines Phosphins und Azodicarbonsäure-diethyl- oder -diisopropylester durchführt.

3. Verfahren gemäß Anspruch 1, dadurch gekennzeichnet, daß man als Mineralsäure wäßrige Salzsäure verwendet.

## Claims

1. A process for the preparation of a 3β-aminocholanic acid derivative of the formula II 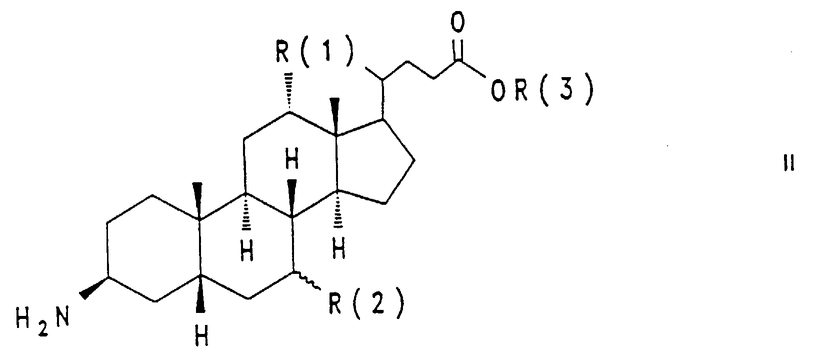 in which
R(1) is H or OH,
R(2) is H, α-OH or β-OH
and
R(3) is an unbranched C₁-C₄-alkyl radical or a branched C₃-C₄-alkyl or a benzyl radical,
or of a salt thereof with a mineral acid,
which comprises
a) reacting a 3α-hydroxycholanic acid ester of the formula III 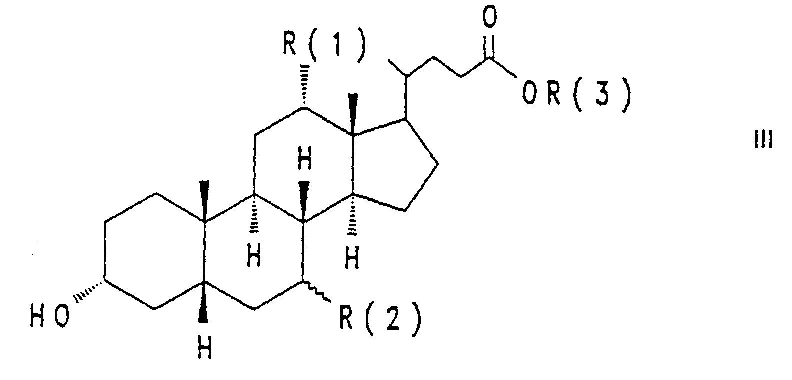 with phthalimide to give the 3β-phthalimido derivative of the formula IV 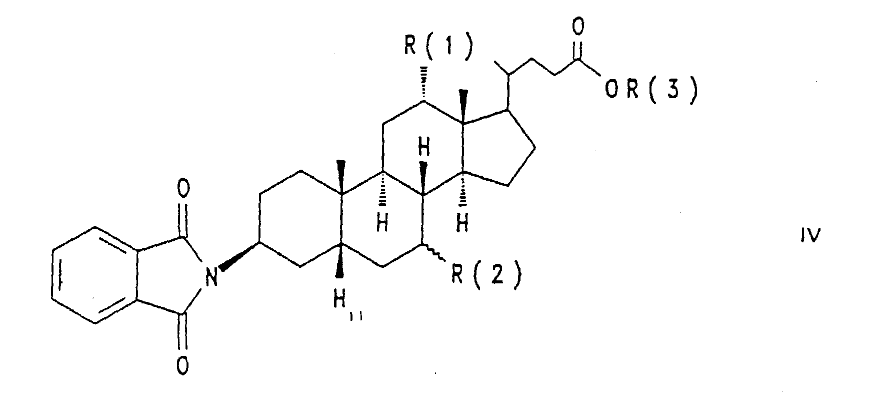
b) reacting a resulting 3β-phthalimidocholanic acid ester of the formula IV with hydrazine hydrate or phenylhydrazine and
c) splitting the phthalimide group by treating the reaction product with an aqueous solution of a mineral acid, an acid addition salt of the compound II with the mineral acid being formed, and
d) if appropriate converting a resulting salt into the free compound of the formula II.

2. The process as claimed in claim 1, wherein the reaction of a compound of the formula III with phthalimide is carried out in the presence of a phosphine and azodicarboxylic acid diethyl or diisopropyl ester.

3. The process as claimed in claim 1, wherein aqueous hydrochloric acid is used as the mineral acid.

## Revendications

1. Procédé de préparation de dérivés de l'acide 3β-aminocholanique de formule II, 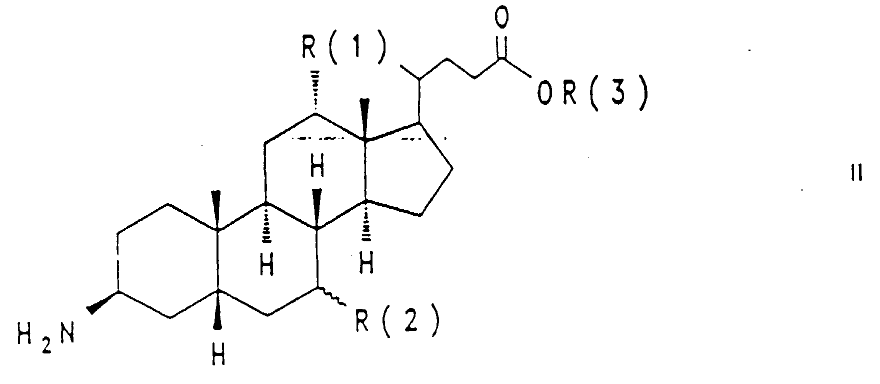 dans laquelle
R(1) = H, OH,
R(2) = H, α-OH ou β-OH
et
R(3) est un radical alkyle en C₁-C₄ non ramifié, un radical alkyle ramifié en C₃-C₄ ou un radical benzyle,
et de leurs sels avec des acides minéraux, caractérisés en ce que
a) on fait réagir un ester de l'acide 3α-hydroxycholanique de formule III 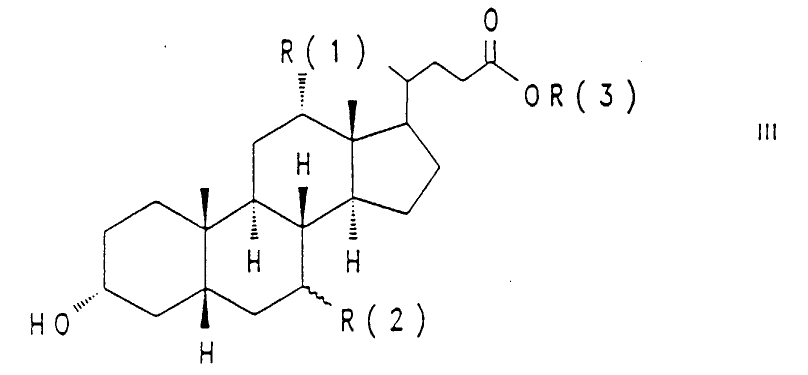 avec le phtalimide pour obtenir le dérivé 3β-phtalimido de formule IV 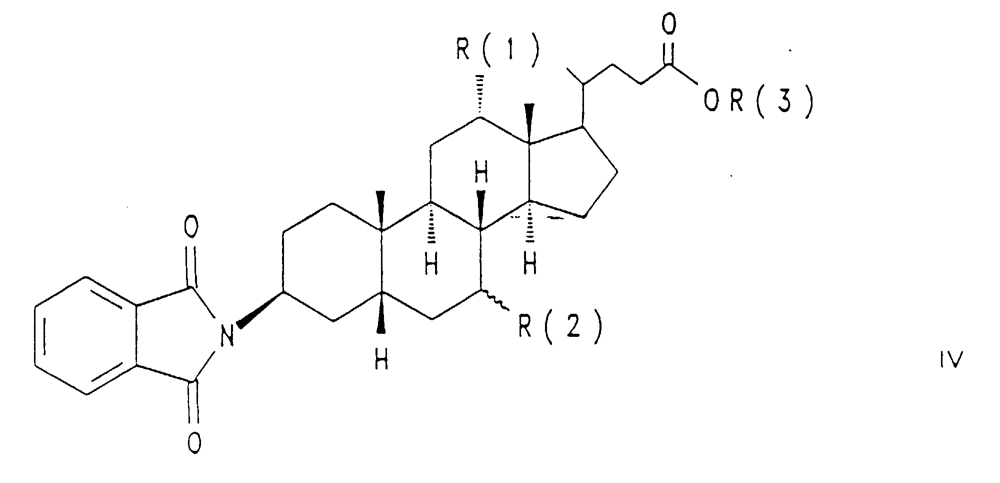
b) on fait réagir un ester de l'acide 3β-phtalimido-cholanique de formule IV obtenu avec de l'hydrate d'hydrazine ou de la phénylhydrazine, et
c) on clive le groupe phtalimide par traitement du produit de la réaction avec une solution aqueuse d'un acide minéral, réaction dans laquelle se forme un sel d'addition avec un acide du composé II avec l'acide minéral, et
d) on transforme éventuellement un sel obtenu en le composé libre.

2. Procédé selon la revendication 1, caractérisé en ce que l'on effectue la réaction des composés de formule III avec du phtalimide en présence d'une phosphine et de l'azodicarboxylate de diéthyle ou de diisopropyle.

3. Procédé selon la revendication 1, caractérisé en ce qu'on utilise comme acide minéral de l'acide chlorhydrique aqueux.
